# EUROPEAN PATENT APPLICATION

(11) **EP 4 606 311 A1**
(43) Date of publication of application: **27.08.2025**
(21) Application number: 25157749.0
(22) Date of filing: 13.02.2025
(51) Int. Cl.: A61B 6/46, A61B 6/00

(54) **RADIOGRAPHY SYSTEM, RADIOGRAPHY METHOD, AND RADIOGRAPHY PROGRAM**

(30) Priority: 26.02.2024 JP 2024026725
(71) Applicant: FUJIFILM Corporation, Tokyo 106-8620 (JP)
(72) Inventor: MAKINO, Kazuhiro, Kasei-machi, 258-8538 (JP); MATSUDA, Hidenori, Kasei-machi, 258-8538 (JP)
(74) Representative: Dehns Germany Partnerschaft mbB

(57) **Abstract**

Provided are a radiography system, a radiography method, and a radiography program that can present a time until next fluoroscopy is able to be started after the end of the fluoroscopy.

A radiography system calculates, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy, and notifies of the calculated remaining time.

## Description

### BACKGROUND OF THE INVENTION

### 1. Field of the Invention

The present disclosure relates to a radiography system, a radiography method, and a radiography program.

### 2. Description of the Related Art

JP2023-000006A discloses a technique of selecting a frame rate of fluoroscopy based on information related to an observation target in an X-ray fluoroscopy apparatus.

### SUMMARY OF THE INVENTION

In fluoroscopy, a storage speed of a video image obtained by fluoroscopy in a storage device may be lower than an imaging speed according to a frame rate of fluoroscopy. In this case, at an end point in time of the fluoroscopy, the storage of the video image in the storage device is not completed, and the storage of the video image in the storage device is continued even after the end of the fluoroscopy. In this case, since a capacity of the video image that cannot be stored in the storage device is increased even in a case in which the next fluoroscopy is started, it is preferable to present the time until the next fluoroscopy can be started after the end of the fluoroscopy to the imaging technician such as an engineer. The fluoroscopy means continuously capturing a plurality of radiation images at a predetermined frame rate (that is, video image capturing).

The present disclosure has been made in view of the above circumstances, and an object of the present disclosure is to provide a radiography system, a radiography method, and a radiography program that can present a time until next fluoroscopy is able to be started after the end of the fluoroscopy.

According to a first aspect, there is provided a radiography system that is capable of executing fluoroscopy in which a plurality of radiation images are continuously captured at a predetermined frame rate, the radiography system comprises at least one processor, in which the processor is configured to calculate, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and notify of the calculated remaining time.

According to a second aspect, in the radiography system according to the first aspect, the processor is configured to, in a case in which the calculated remaining time exceeds a threshold value determined according to a fluoroscopy technique, give a notification.

According to a third aspect, in the radiography system according to the second aspect, the threshold value is determined according to a statistical value of actual values of the remaining time until next fluoroscopy is able to be started in a case in which the fluoroscopy in the past is ended.

According to a fourth aspect, in the radiography system according to the third aspect, the threshold value is determined according to the statistical value of the actual values for each operator of the fluoroscopy.

According to a fifth aspect, in the radiography system according to any one of the first aspect to fourth aspect, the processor is configured to notify of a recommended imaging time according to a fluoroscopy technique before a start of the fluoroscopy.

According to a sixth aspect, in the radiography system according to the fifth aspect, the processor is configured to further notify of a recommended frame rate according to a fluoroscopy technique before a start of the fluoroscopy.

According to a seventh aspect, in the radiography system according to the sixth aspect, the processor is configured to derive the recommended frame rate based on subject information related to a subject to be fluoroscoped and the fluoroscopy technique.

According to an eighth aspect, there is provided a radiography method for a radiography system including at least one processor and capable of executing fluoroscopy in which a plurality of radiation images are continuously captured at a predetermined frame rate, the method comprises calculating, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and notifying of the calculated remaining time.

According to a ninth aspect, there is provided a radiography program for a radiography system including at least one processor and capable of executing fluoroscopy in which a plurality of radiation images are continuously captured at a predetermined frame rate, the radiography program causing the processor to execute processing of: calculating, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and notifying of the calculated remaining time.

According to the present disclosure, it is possible to present a time until next fluoroscopy is able to be started after the end of the fluoroscopy.

### BRIEF DESCRIPTION OF THE DRAWINGS

Fig. 1 is a diagram showing an example of a radiography system.
Fig. 2 is a diagram showing an example of a radiation detector.
Fig. 3 is a block diagram showing an example of a hardware configuration of each device constituting the radiography system.
Fig. 4 is a diagram showing a relationship between a technique of fluoroscopy and an imaging time and the number of times of imaging operations.
Fig. 5 is a diagram for describing a radiation image that has not been stored at an end point in time of the fluoroscopy.
Fig. 6 is a block diagram showing an example of a functional configuration of the console.
Fig. 7 is a diagram showing an example of a notification screen.
Fig. 8 is a sequence diagram showing an example of fluoroscopy processing.
Fig. 9 is a diagram showing an example of a warning notification screen.
Fig. 10 is a diagram showing an example of a recommended frame rate notification screen.
Fig. 11 is a diagram for describing processing of deriving a recommended frame rate.

### DESCRIPTION OF THE PREFERRED EMBODIMENTS

Hereinafter, exemplary embodiments for implementing the technique of the present disclosure will be described in detail with reference to the drawings.

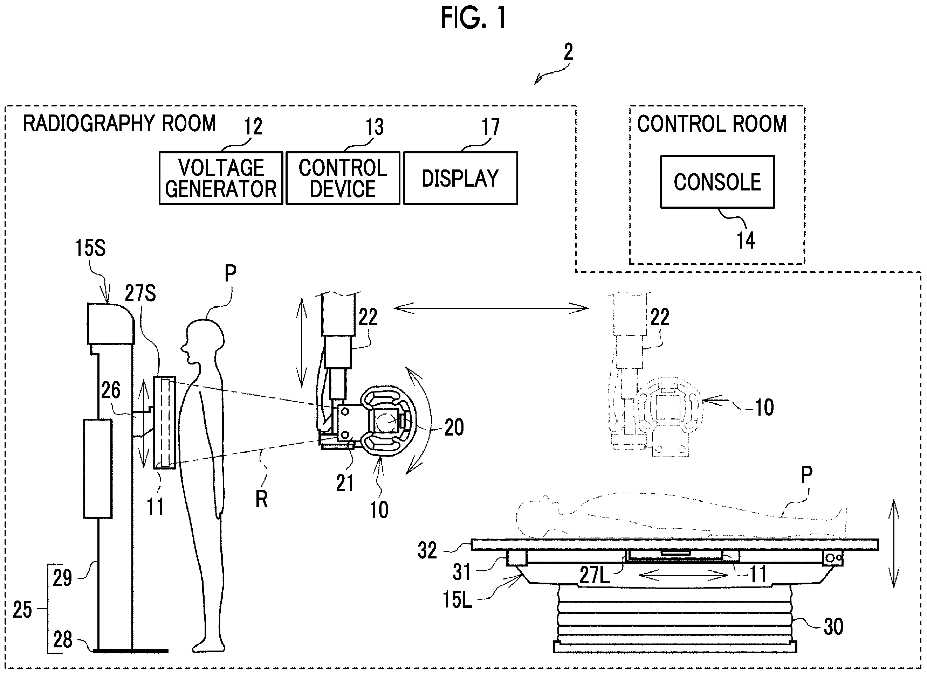

First, a configuration of a radiography system 2 will be described with reference to Fig. 1. As shown in Fig. 1, a radiography system 2 is a system that irradiates a patient P as an example of a subject with radiation R, such as X-rays or γ-rays, and captures a radiation image of the patient P, and is operated by an operator (hereinafter, referred to as an "imaging operator") of radiographic imaging, such as a radiologist. The radiography system 2 is capable of executing fluoroscopy in which a plurality of radiation images are continuously captured at a predetermined frame rate and general imaging that records one radiation image in a switchable manner. The radiography system 2 includes a radiation source 10, a radiation detector 11, a voltage generator 12, a control device 13, a console 14, an upright imaging stand 15S, a decubitus imaging table 15L, and a display 17. The radiation source 10, the radiation detector 11, the voltage generator 12, the control device 13, the upright imaging stand 15S, the decubitus imaging table 15L, and the display 17 are installed in, for example, a radiography room of a medical facility. On the other hand, the console 14 is installed in, for example, a control room adjacent to the radiography room. One radiation source 10 and one radiation detector 11 are prepared, and are used in common in the upright imaging stand 15S and the decubitus imaging table 15L.

The radiation source 10 includes a radiation tube 20 that emits the radiation R and an irradiation field limiter (also referred to as a collimator) 21 that limits an irradiation field of the radiation R. The radiation tube 20 is provided with, for example, a filament, a target, a grid electrode, and the like. A voltage is applied between the filament as a cathode and the target as an anode from the voltage generator 12. The voltage that is applied between the filament and the target is referred to as a tube voltage. The filament releases thermoelectrons according to the applied tube voltage toward the target. The target radiates the radiation R with collision of the thermoelectrons released from the filament. The grid electrode is disposed between the filament and the target. The grid electrode changes a flow rate of the thermoelectrons from the filament toward the target according to the voltage applied from the voltage generator 12. The flow rate of the thermoelectrons from the filament toward the target is referred to as a tube current.

An incident opening through which the radiation R from the radiation tube 20 is incident and an exit opening through which the radiation R exits are formed in the irradiation field limiter 21. Four shielding plates are provided in the vicinity of the exit opening. The shielding plate is formed of a material that shields the radiation R, for example, lead. The shielding plates are each disposed on each side of a quadrangle, in other words, are assembled in a checkered pattern and form a quadrangular irradiation opening through which the radiation R is transmitted. The irradiation field limiter 21 changes a size of the irradiation opening by changing a position of each shielding plate, thereby changing an irradiation field of the radiation R.

The radiation source 10 is suspended from a ceiling of the radiography room by a support column 22. The support column 22 is attached to a rail turned around the ceiling through a wheel. The support column 22 and the radiation source 10 are movable in a horizontal direction in the radiography room by the rail and the wheel. The support column 22 is extendable in a height direction, and accordingly, the radiation source 10 is movable in the height direction. The radiation source 10 is rotatable with respect to the support column 22 with an axis orthogonal to a paper surface as a rotation axis.

The radiation detector 11 is portable, and detects the radiation R transmitted through the patient P to output the radiation image of the patient P. The radiation detector 11 transmits the radiation image to the console 14. The radiation detector 11 is used while being accommodated in the upright imaging stand 15S or the decubitus imaging table 15L. In addition, the radiation detector 11 may be used in a state of being removed from the upright imaging stand 15S or the decubitus imaging table 15L in the radiography room and held by the patient P, or may be used in a state of being placed under the patient P who is lying on a bed in a hospital room. Fig. 1 illustrates an aspect in which the radiation image of the chest of the patient P positioned in front of the upright imaging stand 15S is captured.

The voltage generator 12 generates a tube voltage to be applied to the radiation tube 20. The voltage generator 12 and the radiation tube 20 are connected by a voltage cable. The tube voltage generated by the voltage generator 12 is supplied to the radiation tube 20 through the voltage cable.

The control device 13 controls an operation of the radiation source 10 via the voltage generator 12 according to an irradiation condition of the radiation R. The irradiation condition includes the tube voltage and the tube current applied to the radiation tube 20 and an irradiation time of the radiation R. Further, instead of the tube current and the irradiation time, a product of the tube current and the irradiation time, that is, a so-called mAs value may be used as the irradiation condition. In the fluoroscopy, the control device 13 decides the irradiation condition in the next frame based on a dose of the radiation R that has reached the radiation detector 11, which is derived from the radiation image of the previous frame. As a result, the control device 13 performs dose control of the radiation R in each frame during irradiation with the radiation R in the fluoroscopy.

Each of a radiography start instruction and a radiography end instruction is input by an imaging operator to the control device 13 through an irradiation switch (not shown). The irradiation switch is installed in at least one of the control room or the radiography room. The irradiation switch may be a switch operated by a hand or a switch operated by a foot. In a case where the start instruction is input, the control device 13 operates the voltage generator 12 according to the irradiation condition to emit the radiation R from the radiation tube 20.

The console 14 has a function of allowing the imaging operator to confirm and input irradiation conditions of the radiation R, and a function of performing image processing on the radiation image obtained by the radiation detector 11. Examples of the console 14 include a computer such as a personal computer or a server computer.

The upright imaging stand 15S includes a stand 25, a connection portion 26, the upright posture holder 27S, and the like. The stand 25 is configured with a pedestal 28 that is installed on a floor surface of the radiography room, and a support column 29 that extends from the pedestal 28 in the height direction. The connection portion 26 connects the upright posture holder 27S to the stand 25. The connection portion 26 and the upright posture holder 27S are movable in the height direction with respect to the support column 29 and can perform height adjustment according to the height of the patient P or an imaging site.

The upright posture holder 27S has a box shape and accommodates the radiation detector 11 therein. The upright posture holder 27S is mostly formed of a conductive material having an electromagnetic wave shielding property, such as aluminum or stainless steel. The upright posture holder 27S has a front surface facing the radiation source 10, and the front surface is formed of a material transmitting the radiation R, such as carbon.

The decubitus imaging table 15L has a pedestal 30 that is installed on the floor surface of the radiography room, a connection portion 31, a top plate 32, the decubitus posture holder 27L, and the like. The connection portion 31 connects the top plate 32 to the pedestal 30. The pedestal 30 is of an elevating type, and thus heights of the top plate 32 and the decubitus posture holder 27L can be adjusted. The top plate 32 has a rectangular plate shape having a length and a width such that the patient P can lie, and is formed of a material transmitting the radiation R, such as carbon.

The decubitus posture holder 27L is disposed in a space between the pedestal 30 and the top plate 32 formed by the connection portion 31. The decubitus posture holder 27L has a box shape with a top covered with the top plate 32, and accommodates the radiation detector 11 therein. The decubitus posture holder 27L is formed of a conductive material having an electromagnetic wave shielding property, such as aluminum or stainless steel. The decubitus posture holder 27L is slidable in a direction along a longitudinal direction of the top plate 32 by a sliding mechanism.

The display 17 is a liquid crystal display or an electro luminescence (EL) display. The display 17 is installed on a display cart with a caster and is movable in the radiography room. In addition, the display 17 may be a liquid crystal display disposed in the radiation source 10. In addition, the display 17 may be a liquid crystal display disposed in the irradiation field limiter 21. The display 17 is connected to the console 14.

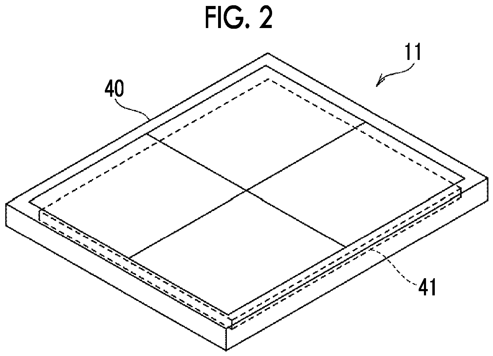

As shown in Fig. 2, the radiation detector 11 has a housing 40 and a detection panel 41. The housing 40 has a flat, generally rectangular parallelepiped shape with a rectangular plane shape, and houses the detection panel 41 therein. Most of a front surface of the housing 40 is formed of a material transmitting the radiation R, such as carbon. The radiation detector 11 is set in the upright posture holder 27S or the decubitus posture holder 27L in a posture in which the front surface of the housing 40 faces the radiation source 10.

The detection panel 41 has a configuration in which a plurality of pixels that are sensitive to the radiation R or visible light converted from the radiation R by a scintillator to generate signal charges are arranged. In addition to the detection panel 41, a control device 18 described below is incorporated in the housing 40. In addition, a communication unit, a battery that supplies power to each unit, and the like are also incorporated in the housing 40. The radiation detector 11 may be a so-called computed radiography (CR) cassette in which an imaging plate is incorporated in place of the detection panel 41.

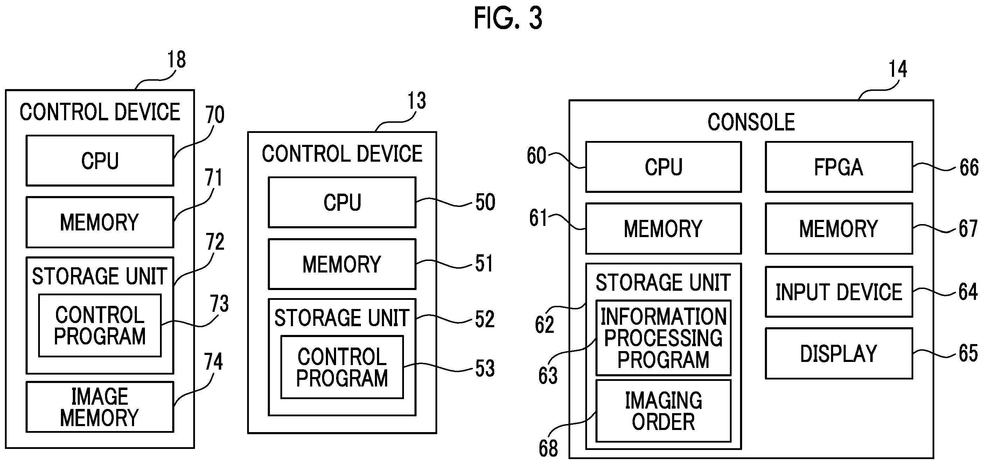

Next, a hardware configuration of the control device 13, the console 14, and the control device 18 will be described with reference to Fig. 3. As shown in Fig. 3, the control device 13 includes a central processing unit (CPU) 50, a memory 51 as a temporary storage area, and a non-volatile storage unit 52. The CPU 50 is an example of a processor.

The storage unit 52 is realized by a hard disk drive (HDD), a solid state drive (SSD), a flash memory, or the like. The storage unit 52 as a storage medium stores a control program 53. The CPU 50 reads out the control program 53 from the storage unit 52, loads the control program 53 into the memory 51, and executes the loaded control program 53.

The console 14 includes a CPU 60, a memory 61 used as a temporary storage area by the CPU 60, a non-volatile storage unit 62, an input device 64 such as a keyboard and a mouse, and a display 65 such as a liquid crystal display or an EL display. In addition, the console 14 further includes a field programmable gate array (FPGA) 66 and a memory 67 used as a temporary storage area by the FPGA 66. The CPU 60 and the FPGA 66 are examples of a processor. The FPGA 66 includes a logic circuit in which logic of image processing performed on the radiation image is programmed in advance.

The storage unit 62 is realized by an HDD, an SSD, a flash memory, or the like. An information processing program 63 is stored in the storage unit 62 as a storage medium. The CPU 60 reads out the information processing program 63 from the storage unit 62, loads the information processing program 63 into the memory 61, and executes the loaded information processing program 63. The information processing program 63 is an example of a radiography program according to the disclosed technology.

In addition, the storage unit 62 stores the imaging order 68 that is registered in advance. The imaging order 68 includes irradiation conditions of the radiation R as an example of the imaging conditions of the radiation image in the fluoroscopy. In addition, the imaging order 68 includes patient information such as an age of the patient P, information on a physique of the patient P, and the imaging site. The storage unit 62 is an example of a storage device according to the disclosed technology.

The control device 18 includes a CPU 70, a memory 71 as a temporary storage area, a non-volatile storage unit 72, and an image memory 74. The CPU 70 is an example of a processor. The storage unit 72 is realized by an HDD, an SSD, a flash memory, or the like. The storage unit 72 as a storage medium stores a control program 73. The CPU 70 reads out the control program 73 from the storage unit 72, loads the control program 73 into the memory 71, and executes the loaded control program 73. The image memory 74 has a storage capacity capable of storing a predetermined number of radiation images.

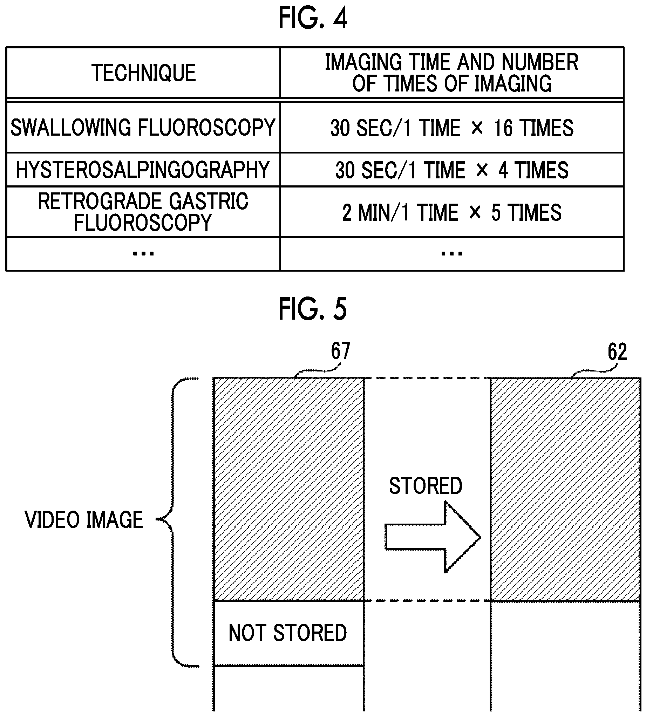

By the way, as shown in Fig. 4, in the fluoroscopy, the imaging time of single fluoroscopy and the number of times of fluoroscopy in single examination vary depending on the fluoroscopy technique. In addition, in the fluoroscopy, the time from the completion of single fluoroscopy to the start of the next single fluoroscopy also varies depending on the fluoroscopy technique. For example, in a case of a technique in which it is not necessary to change the positioning of the patient P and the irradiation conditions of the radiation R, the time until the next single fluoroscopy can be started after the completion of the single fluoroscopy is relatively short. The single fluoroscopy means imaging from the imaging operator's instruction to start imaging through the irradiation switch to the imaging operator's instruction to end imaging. That is, it means that there is a technique in which a plurality of times of fluoroscopy are performed in single examination.

In addition, in the fluoroscopy, the FPGA 66 performs various types of image processing, such as offset correction processing, sensitivity correction processing, and defective pixel correction processing, on the radiation image of each frame, and the radiation image after the image processing is temporarily stored in the memory 67. In addition, the radiation image stored in the memory 67 is permanently stored by being stored in the storage unit 62. The radiation image stored in the memory 67 is displayed on the display 17, so that the imaging operator can confirm the radiation image of each frame.

In general, a data storage speed in the memory 67 is higher than a data storage speed in the storage unit 62. That is, in the fluoroscopy, the storage speed in the storage unit 62 is lower than the imaging speed of the radiation image. The imaging speed of the radiation image means a speed until the radiation detector 11 acquires the radiation image, the FPGA 66 performs various types of image processing on the radiation image, and the radiation image is stored in the memory 67, that is, until the radiation image is displayed on the display 17 and can be confirmed by the imaging operator. In addition, the storage speed of the radiation image in the storage unit 62 means a speed of storing the radiation image stored in the memory 67 in the storage unit 62. In the present embodiment, the units of the imaging speed and the storage speed are expressed in frame per second (fps).

Therefore, as shown in Fig. 5, at the end point in time of single fluoroscopy, there is a radiation image whose storage in the storage unit 62 has not been completed in the radiation images of each frame constituting the video image stored in the memory 67 by the fluoroscopy. In the example of Fig. 5, a portion filled with diagonal lines represents a storage region of the radiation image whose storage in the storage unit 62 has been completed at the end point in time of the fluoroscopy. In this case, it is preferable that the next single fluoroscopy is performed after the storage of the radiation images of all the frames constituting the video image in the storage unit 62 is completed. Therefore, the console 14 according to the present embodiment has a function of notifying of the remaining time until the storage of the video image is completed in a case in which the fluoroscopy is ended. The video image referred to here refers to video image data.

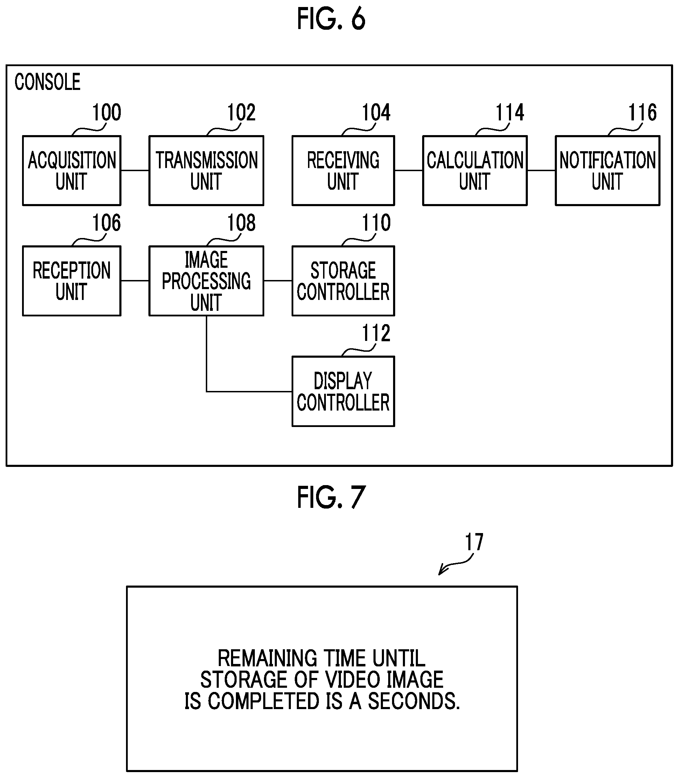

Next, a functional configuration of the console 14 will be described with reference to Fig. 6. As shown in Fig. 6, the console 14 includes an acquisition unit 100, a transmission unit 102, a receiving unit 104, a reception unit 106, an image processing unit 108, a storage controller 110, a display controller 112, a calculation unit 114, and a notification unit 116. The CPU 60 executes the information processing program 63 to function as the acquisition unit 100, the transmission unit 102, the receiving unit 104, the reception unit 106, the storage controller 110, the display controller 112, the calculation unit 114, and the notification unit 116. The FPGA 66 executes a logic programmed in advance to function as the image processing unit 108.

The acquisition unit 100 acquires the imaging order 68 from the storage unit 62. The transmission unit 102 transmits the irradiation condition in the fluoroscopy corresponding to the imaging order 68 acquired by the acquisition unit 100 to the control device 13.

The receiving unit 104 receives an instruction to start fluoroscopy input by the imaging operator through the irradiation switch. In addition, the receiving unit 104 receives an instruction to end fluoroscopy input by the imaging operator through the irradiation switch.

The reception unit 106 receives a video image obtained by fluoroscopy, which is the video image transmitted from the radiation detector 11. In the present embodiment, the radiation images constituting the video image are transmitted from the radiation detector 11 to the console 14 one frame at a time. The reception unit 106 receives the radiation images constituting the video image one frame at a time.

The image processing unit 108 performs various types of image processing, such as offset correction processing, sensitivity correction processing, and defective pixel correction processing, on the video image received by the reception unit 106. The image processing unit 108 performs image processing on the radiation image constituting the video image for each frame and stores the radiation image on which the image processing is completed in the memory 67.

The storage controller 110 stores the video image in the storage unit 62 by sequentially storing the radiation image of each frame constituting the video image stored in the memory 67 in the storage unit 62.

The display controller 112 displays the video image on the display 17 by performing control of sequentially displaying the radiation image of each frame constituting the video image stored in the memory 67 on the display 17. The display controller 112 may perform control to display the irradiation conditions of the radiation R and the patient information related to the fluoroscopy on the display 65 based on the imaging order 68. In addition, in a case where the display 17 comprises a plurality of panels, the display controller 112 may perform control to display a video image obtained by fluoroscopy on the first panel and control to display the same screen as the display 65 on the second panel.

In a case in which the instruction to end the fluoroscopy is received by the receiving unit 104, that is, in a case in which the fluoroscopy is ended, the calculation unit 114 calculates the remaining time (hereinafter, simply referred to as a "remaining time") until the storage of the video image obtained by the fluoroscopy in the storage unit 62 is completed. In this case, the calculation unit 114 calculates the remaining time based on the data size of the video image, whose storage in the storage unit 62 has been completed, among the video images temporarily stored in the memory 67 by the fluoroscopy, the data storage speed in the storage unit 62, and the data size of the video image obtained by the fluoroscopy according to the frame rate and the imaging time of the fluoroscopy.

Specifically, the calculation unit 114 calculates the remaining time according to the following Equation (1). Remaining time = (data size of video image obtained by fluoroscopy - data size of video image whose storage in storage unit 62 is completed) ÷ data storage speed in storage unit 62

The data size obtained by the subtraction in the parentheses on the right side of Equation (1) is the data size of the radiation image whose storage in the storage unit 62 has not been completed at the point in time when the fluoroscopy is ended, as shown in Fig. 5.

For example, in a case in which the imaging speed of the fluoroscopy is 15 [fps], the imaging time of the fluoroscopy is 60 [sec], and the data storage speed in the storage unit 62 is 10 fps, the data size of the radiation image whose storage in the storage unit 62 is not completed at the end point in time of the fluoroscopy is obtained by the following Equation (2). Data size of radiation image of one frame × 15 [fps] × 60 [sec] - data size of radiation image of one frame × 10 [fps] × 60 = data size of image of one frame × 5 [fps] × 60

Further, the remaining time in this case is obtained by the following Equation (3). (data size of radiation image of one frame × 5 [fps] × 60) ÷ (data size of radiation image of one frame × 10 [fps]) = 30 [sec]

The data storage speed in the storage unit 62 may be set in advance based on the performance evaluation result of the shipment inspection of the console 14. In addition, the data storage speed in the storage unit 62 may be set by the self-check performed by the CPU 60 at the time of the activation of the console 14. In addition, the calculation unit 114 may calculate the data storage speed in the storage unit 62 by dividing the data size of the video image whose storage in the storage unit 62 is completed by the time required for the storage in the storage unit 62, during the implementation of the fluoroscopy. In addition, the calculation unit 114 may calculate the data storage speed in the storage unit 62 by dividing the data size of the video image obtained by the latest fluoroscopy by the time required for the storage in the storage unit 62.

The notification unit 116 notifies the remaining time calculated by the calculation unit 114. In the present embodiment, as shown in Fig. 7 as an example, the notification unit 116 performs control to display the remaining time on the display 17 to notify of the imaging operator of the remaining time. The notification unit 116 may perform control to display the remaining time on the display 17 and then perform control to count down and display the remaining time as time elapses.

In addition, the notification unit 116 may notify of the remaining time by voice through a voice output device such as a speaker. In this case, the notification unit 116 may notify of the remaining time by a predetermined sound such as a beep sound. For example, the notification unit 116 may notify of the remaining time by shortening the sound interval of the beep sound as the remaining time is shorter. In addition, the notification unit 116 may notify of the remaining time by increasing the volume of the beep sound as the remaining time is shorter.

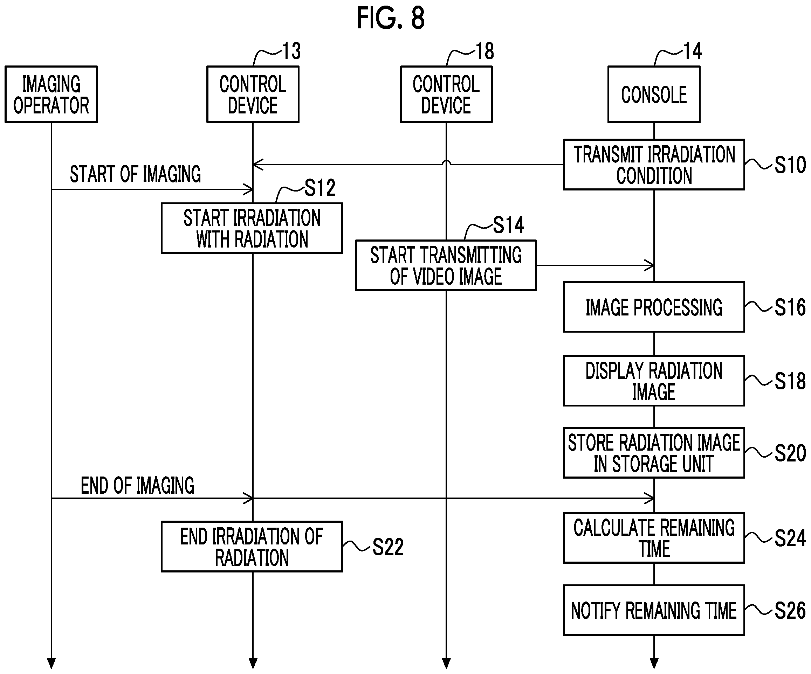

Next, an action of the radiography system 2 will be described with reference to Fig. 8. Fig. 8 is a sequence diagram showing an example of fluoroscopy processing executed by the radiography system 2.

In a case where the imaging order 68 is registered, in step S10, the acquisition unit 100 acquires the imaging order 68 from the storage unit 62. Then, the transmission unit 102 transmits the irradiation condition in the fluoroscopy corresponding to the imaging order 68 acquired by the acquisition unit 100 to the control device 13.

The imaging operator makes the patient P stand in front of the upright imaging stand 15S or makes the patient P lie on the top plate 32 of the decubitus imaging table 15L according to the imaging order 68. Then, the imaging operator inputs an instruction to start fluoroscopy through the irradiation switch. In a case in which the instruction to start the fluoroscopy is input, in step S12, the CPU 50 of the control device 13 operates the voltage generator 12 according to the irradiation conditions in the fluoroscopy transmitted from the console 14 in step S10, and emits the radiation R from the radiation tube 20. As a result, the irradiation with the radiation R is started.

In step S14, the CPU 70 of the control device 18 starts the transmission of the video image obtained at a predetermined frame rate. As a result, the CPU 70 sequentially transmits the radiation image of each frame constituting the video image obtained by the fluoroscopy to the console 14. The reception unit 106 sequentially receives the radiation images transmitted from the radiation detector 11 in step S14.

In step S16, as described above, the image processing unit 108 performs various types of image processing on the radiation image received by the reception unit 106 and stores the radiation image in which the image processing is completed in the memory 67. In step S18, the display controller 112 performs control to display the radiation image stored in the memory 67 in step S16 on the display 17. By repeatedly executing the processing of step S16 and step S18 in accordance with a predetermined frame rate from the start to the end of the fluoroscopy, the video image obtained by the fluoroscopy is displayed on the display 17.

In step S20, by sequentially storing the radiation image of each frame constituting the video image stored in the memory 67 in step S18 in the storage unit 62, the video image is stored in the storage unit 62. The processing of step S20 is executed from the start of the fluoroscopy until the storage of the radiation images of all frames constituting the video image obtained by the fluoroscopy in the storage unit 62 is completed.

In a case in which single fluoroscopy is completed, the imaging operator inputs an instruction to end the fluoroscopy through the irradiation switch. In a case in which the instruction to end the fluoroscopy is input, in step S22, the CPU 50 of the control device 13 is ended the irradiation of the radiation R.

In addition, in a case in which the instruction to end the fluoroscopy is received by the receiving unit 104, in Step S24, the calculation unit 114 calculates the remaining time according to Equation (1) as described above. In step S26, the notification unit 116 notifies the remaining time calculated in step S24 as described above. The imaging operator grasps the notified remaining time and prepares for the next fluoroscopy. In the next fluoroscopy, the fluoroscopy processing of the same sequence is also executed.

As described above, according to the present embodiment, it is possible to present a time until the next fluoroscopy is able to be started after the end of the fluoroscopy.

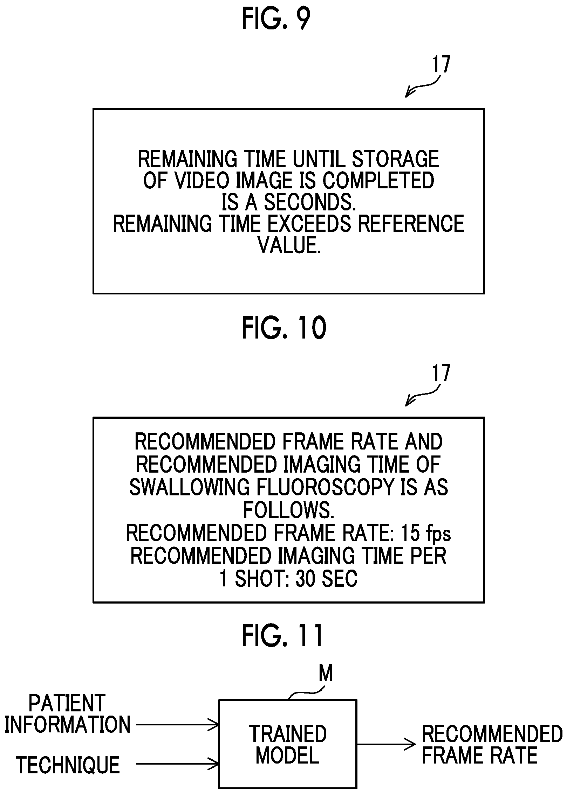

In the above-described embodiment, in a case in which the remaining time calculated by the calculation unit 114 exceeds a threshold value determined according to the fluoroscopy technique, the notification unit 116 may notify of a warning. As shown in Fig. 9 as an example, the notification unit 116 may perform control to display a message indicating that the remaining time exceeds a threshold value on the display 17 in addition to the remaining time, thereby notifying of a warning. In addition, the notification unit 116 may notify of the warning by voice through a voice output device.

In addition, the notification unit 116 may determine the threshold value in this form example according to a statistical value of the actual value of the remaining time until the next fluoroscopy is able to be started in a case in which the fluoroscopy in the past is ended. Examples of the statistical value in this case include an average value and a median value. The actual value of the remaining time referred to here is, for example, the remaining time calculated by the calculation unit 114 in the past fluoroscopy.

In addition, the notification unit 116 may determine the threshold value in this form example according to the statistical value of the actual value for each imaging operator. An example of the statistical value in this case also includes the average value or the median value.

In addition, in the above-described embodiment, the notification unit 116 may notify of a recommended imaging time according to the fluoroscopy technique before the start of the fluoroscopy. In addition, the notification unit 116 may further notify of a recommended frame rate according to the fluoroscopy technique before the start of the fluoroscopy. As shown in Fig. 10 as an example, the notification unit 116 may notify of the recommended imaging time and the recommended frame rate by performing control to display the recommended imaging time and the recommended frame rate according to the fluoroscopy technique on the display 17. Examples of the time before the start of the fluoroscopy include a timing at which the imaging order 68 is registered, a timing at which the imaging operator enters the radiography room, and the like. In addition, the notification unit 116 may notify of the recommended imaging time and the recommended frame rate by voice through the voice output device.

In addition, the CPU 60 may derive the recommended frame rate based on subject information related to a subject to be fluoroscoped and the fluoroscopy technique. In this case, the CPU 60 may use the trained model M obtained by machine learning, which receives the subject information and the technique as inputs and outputs the recommended frame rate. Specifically, as shown in Fig. 11, the CPU 60 may derive the recommended frame rate by inputting patient information and the fluoroscopy technique included in the imaging order 68 to the trained model M.

In addition, at least one of the functional units provided in the console 14 in the above-described embodiment may be provided in the control device 13 or the control device 18.

In addition, in the above-described embodiment, for example, various processors shown below can be used as a hardware structure of a processing unit that executes various types of processing, such as each functional unit of the console 14. The various processors include, in addition to a CPU that is a general-purpose processor functioning as various processing units by executing software (program) as described above, a programmable logic device (PLD) that is a processor whose circuit configuration can be changed after manufacture such as an FPGA, a dedicated electric circuit that is a processor having a circuit configuration dedicatedly designed to execute specific processing such as an application specific integrated circuit (ASIC), and the like.

One processing unit may be composed of one of the various processors or may be composed of a combination of two or more processors of the same type or different types (for example, a combination of a plurality of FPGAs or a combination of a CPU and an FPGA). In addition, a plurality of processing units may be composed of one processor.

As an example of the plurality of processing units composed of one processor, first, as represented by a computer such as a client and a server, a form of one processor that is composed of a combination of one or more CPUs and software and that functions as the plurality of processing units is possible. Second, as represented by a system on chip (SoC) or the like, there is a form in which a processor that realizes functions of an entire system including a plurality of processing units with one integrated circuit (IC) chip is used. Accordingly, various processing units are configured using one or more of the various processors as the hardware structure.

Furthermore, more specifically, an electric circuit (circuitry) in which circuit elements such as semiconductor elements are combined can be used as the hardware structure of the various processors.

In the above-described embodiment, an aspect in which various programs are stored (installed) in the storage unit in advance has been described, but the present disclosure is not limited thereto. The various programs may be provided in a form of being recorded on a recording medium such as a compact disc read only memory (CD-ROM), a digital versatile disc read only memory (DVD-ROM), and a Universal Serial Bus (USB) memory. The various programs may be provided in a form of being downloaded from an external device via a network.

### Explanation of References

2: radiography system
10: radiation source
11: radiation detector
12: voltage generator
13, 18: control device
14: console
15L: decubitus imaging table
15S: upright imaging stand
17, 65: display
20: radiation tube
21: irradiation field limiter
22, 29: support column
25: stand
26, 31: connection portion
27L: decubitus posture holder
27S: upright posture holder
28, 30: pedestal
32: top plate
40: housing
41: detection panel
50, 60, 70: CPU
51, 61, 67, 71: memory
52, 62, 72: storage unit
53, 73: control program
63: information processing program
64: input device
66: FPGA
68: imaging order
74: image memory
100: acquisition unit
102: transmission unit
104: receiving unit
106: reception unit
108: image processing unit
110: storage controller
112: display controller
114: calculation unit
116: notification unit
M: trained model
P: patient
R: radiation

## Claims

1. A radiography system that is capable of executing fluoroscopy in which a plurality of radiation images are continuously captured at a predetermined frame rate, the radiography system comprising at least one processor,
wherein the processor is configured to:
calculate, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and
notify of the calculated remaining time.

2. The radiography system according to claim 1,
wherein the processor is configured to, in a case in which the calculated remaining time exceeds a threshold value determined according to a fluoroscopy technique, give a notification.

3. The radiography system according to claim 2,
wherein the threshold value is determined according to a statistical value of actual values of the remaining time until next fluoroscopy is able to be started in a case in which the fluoroscopy in the past is ended.

4. The radiography system according to claim 3,
wherein the threshold value is determined according to the statistical value of the actual values for each operator of the fluoroscopy.

5. The radiography system according to any one of claims 1 to 4,
wherein the processor is configured to notify of a recommended imaging time according to a fluoroscopy technique before a start of the fluoroscopy.

6. The radiography system according to claim 5,
wherein the processor is configured to further notify of a recommended frame rate according to the fluoroscopy technique before the start of fluoroscopy.

7. The radiography system according to claim 6,
wherein the processor is configured to derive the recommended frame rate based on subject information related to a subject to be fluoroscoped and the fluoroscopy technique.

8. A radiography method for a radiography system including at least one processor and capable of executing fluoroscopy in which a plurality of radiation images are continuously captured at a predetermined frame rate, the method comprising:
calculating, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and
notifying of the calculated remaining time.

9. A radiography program for a radiography system including at least one processor and capable of executing fluoroscopy in which a plurality of radiation images are continuously captured at a predetermined frame rate, the radiography program causing the processor to execute processing of:
calculating, in a case in which the fluoroscopy is ended, a remaining time until storage of a video image obtained by the fluoroscopy in a storage device is completed, based on a data size of the video image for which the storage in the storage device is completed among video images temporarily stored in a memory by the fluoroscopy, a data storage speed in the storage device, and a data size of the video image obtained by the fluoroscopy according to a frame rate and an imaging time of the fluoroscopy; and
notifying of the calculated remaining time.
